# EUROPEAN PATENT APPLICATION

(11) **EP 4 477 229 A1**
(43) Date of publication of application: **18.12.2024**
(21) Application number: 22911468.1
(22) Date of filing: 16.05.2022
(51) Int. Cl.: A61K 38/16, A61P 27/02, A23L 33/195, A23K 20/189, C12N 9/02, C12N 15/75

(54) **SUPEROXIDE DISMUTASE AND USES THEREOF FOR PREVENTING OR TREATING DRY MACULAR DEGENERATION**

(30) Priority: 23.12.2021 KR 20210185912
(71) Applicant: Genofocus, Inc., Daejeon 34014 (KR); BiomLogic, Inc., Seoul 03923 (KR)
(72) Inventor: PAN, Jae Gu, Seoul 03923 (KR); KIM, Eui Joong, Daejeon 34014 (KR); CHANG, In Ik, Seoul 03923 (KR); KIM, Jeong Hyun, Daejeon 34014 (KR)
(74) Representative: Hoefer & Partner Patentanwälte mbB
(86) International application number: PCT/KR2022/007001
(87) International publication number: WO 2023/120828

(57) **Abstract**

The present invention relates to superoxide dismutase and a use thereof for preventing or treating dry age-related macular degeneration. More specifically, the present invention relates to a pharmaceutical composition or a therapeutic method for prevention or treatment of dry macular degeneration. The composition and method according to the present invention can be effectively used to prevent or treat dry macular degeneration by reducing or preventing drusen formation and/or drusenoid lesions.

## Description

### Technical Field

The present disclosure relates to a superoxide dismutase and a use thereof for preventing, ameliorating, or treating dry macular degeneration. More specifically, the present disclosure relates to a superoxide dismutase, a composition, or a method for preventing, treating, or ameliorating dry macular degeneration.

### Background Art

Macular degeneration, also called age-related macular degeneration ("AMD"), refers to a chronic, progressive degenerative pathology of the macula, which results in loss of central vision. Macular degeneration is a leading cause of vision loss and irreversible central vision loss in adults over 50 years of age. More than 25 million people around the world suffer from macular degeneration, and the number of patients with macular degeneration continues to increase rapidly due to the rapid growth of the elderly population. In addition, excessive use of electronic devices such as smartphones and laptops causes the early onset and increased prevalence of macular degeneration in people today.

The most important causes of macular degeneration are age-related atrophy and a decline in the function of retinal pigment epithelium (RPE). The retinal pigment epithelium plays a critical role in maintaining the homeostasis and physiological function of the retina that plays a key role in visual function. In addition, the age-related abnormal changes in Bruch's membrane and degeneration of choroidal capillaries are also thought to contribute to the etiology of macular degeneration. Bruch's membrane functions as the basement membrane of the RPE, while choroidal capillaries are located on the outermost side of the neural retina and supply nutrients and oxygen to photoreceptor cells in which photoconversion occurs. It is known that excessive free radicals, mitochondrial dysfunction, inflammation, abnormality in lipid metabolism, genetic factors, and environmental factors (for example, smoking, long-term exposure to ultraviolet light) play a role in the pathogenesis of macular degeneration; however, the exact mechanism is not yet fully known.

Macular degeneration is largely classified into two types: wet and dry. The dry type accounts for 85 to 90% of cases diagnosed with macular degeneration, and the wet type also originates from the dry type. The dry macular degeneration is primarily characterized by medium-sized or larger yellow deposits, called drusen, which exist beneath the retinal pigment epithelium layer, pigmentary abnormalities of the retinal pigment epithelium, and deposits, called reticular pseudodrusen, which exist beneath the retina. Formation of drusen is a typical feature of dry macular degeneration, and presence of multiple medium- and large-sized drusens is known to be a risk factor for progression to end-stage macular degeneration. In addition, the size and density of drusen are correlated with loss of choriocapillaris.

Dry macular degeneration generally progresses slowly and, in its later stages, may progress to geographic atrophy (GA). Geographic atrophy causes confluent or scattered irreversible degeneration of the retinal pigment epithelium, which damages photoreceptors. If geographic atrophy continues, it may lead to loss of vision. Geographic atrophy is a major cause of vision impairment due to aging; however, there are currently no approved treatments or medications that can delay or inhibit progression of dry macular degeneration.

In wet macular degeneration, which is another type of end-stage macular degeneration, immature blood vessels grow from the choroid toward the retina, and this causes blood or body fluid to leak while damaging photoreceptors and the nerve cells connected thereto, thereby ultimately resulting in loss of central vision. Wet macular degeneration progresses faster than the dry type, and thus may lead to blindness within a few months if left untreated. However, as an anti-angiogenic therapy to prevent formation of new blood vessels, a treatment method has been developed in which an anti-VEGF agent is injected into the eye to inhibit abnormally produced VEGF so that the angiogenesis process is stopped.

Therefore, there is a need for materials and methods which can effectively prevent or treat dry macular degeneration.

### Disclosure of Invention

### Technical Problem

The object of the present disclosure is to solve the above-mentioned problems of the prior art.

Another object of the present disclosure is to provide a superoxide dismutase for preventing, ameliorating, or treating dry macular degeneration.

Yet another object of the present disclosure is to provide a pharmaceutical composition or treatment method for preventing or treating dry macular degeneration.

Still yet another object of the present disclosure is to provide a food or feed composition for preventing or ameliorating dry macular degeneration.

Still yet another object of the present disclosure is to provide a pharmaceutical composition or treatment method for preventing or treating dry macular degeneration by reducing or eliminating formation of drusen or drusenoid lesions.

Still yet another object of the present disclosure is to provide a food or feed composition for preventing or ameliorating dry macular degeneration by reducing or eliminating formation of drusen or drusenoid lesions.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided an improved superoxide dismutase (SOD) that exhibits an effect of preventing, ameliorating, or treating dry macular degeneration.

According to another aspect of the present disclosure, there is provided a superoxide dismutase derived from a generally regarded as safe (GRAS) bacterium, such as Bacillus, for preventing, ameliorating, or treating dry macular degeneration.

According to yet another aspect of the present disclosure, there is provided a superoxide dismutase, which is derived from *Bacillus amyloliquefaciens* and for which oral administration efficacy and safety are secured, for preventing, ameliorating, or treating dry macular degeneration.

According to still yet another aspect of the present disclosure, there is provided a superoxide dismutase, which is derived from Bacillus or *Bacillus amyloliquefaciens* and is secreted extracellularly for convenient production, for preventing or treating dry macular degeneration.

According to still yet another aspect of the present disclosure, there is provided a pharmaceutical composition for preventing or treating dry macular degeneration, comprising the superoxide dismutase as an active ingredient.

According to still yet another aspect of the present disclosure, there is provided a food or feed composition for ameliorating or preventing dry macular degeneration, comprising the superoxide dismutase as an active ingredient.

According to still yet another aspect of the present disclosure, there is provided a method for preventing, ameliorating, or treating dry macular degeneration, comprising administering to a subject the superoxide dismutase.

According to still yet another aspect of the present disclosure, there is provided a use of the superoxide dismutase for preventing or treating dry macular degeneration.

According to still yet another aspect of the present disclosure, there is provided a use of the superoxide dismutase (SOD) in manufacture of a medicament for prevention or treatment of dry macular degeneration.

According to still yet another aspect of the present disclosure, there are provided a superoxide dismutase, which is derived from a Bacillus species strain and whose amino acid residues 73 and 136 are substituted with Asp residues, with respect to SEQ ID NO: 2, a polynucleotide sequence encoding the same, an expression vector comprising the polynucleotide sequence, or a cell line transformed with the expression vector.

According to still yet another aspect of the present disclosure, there is provided a Bacillus species strain which comprises the polynucleotide sequence and in which some genes are deleted.

### Advantageous Effects of Invention

The superoxide dismutase, composition, and method according to the present disclosure can be effectively used to prevent, ameliorate, or treat dry macular degeneration by reducing or preventing formation of drusen and/or drusenoid lesions. In particular, it has been identified that the superoxide dismutase according to the present disclosure is effective in preventing or treating dry macular degeneration in a case of being administered orally.

The superoxide dismutase according to the present disclosure is derived from Bacillus or *Bacillus amyloliquefaciens* which is a generally regarded as safe (GRAS) bacterium. Thus, for the superoxide dismutase, not only can oral administration efficacy and safety be secured, but also production advantage can be taken by being directly recoverable from the supernatant during culture.

A polynucleotide sequence encoding the superoxide dismutase according to the present disclosure, an expression vector comprising the sequence, or a cell line and a Bacillus species strain, each of which is transformed with the expression vector, can be used to effectively produce an active ingredient for reducing or preventing formation of drusen and/or drusenoid lesions.

### Brief Description of Drawings

FIG. 1 illustrates a schematic diagram for the double crossover recombination performed in Example 1.1.
FIG. 2 illustrates results obtained by identifying defective genes using PCR in the expression host GFBS220 and the production strain BSBA310. W represents wild-type *B*. *subtilis* KCTC 3135, 220 represents the expression host GFBS220, and 310 represents the SodA2 production strain BSBA310.
FIG. 3 illustrates comparison of the amino acid sequences between SodA (Mn-SOD identified through N-terminal sequencing from the culture supernatant of *Bacillus amyloliquesfaciens* strain GF423) and the superoxide dismutase (SodA2) of the present disclosure.
FIG. 4 illustrates an expression vector for overexpression of *sodA2* gene. Here, rrnB T1T2 represents a transcription terminator; rep(pBR322) represents a pBR322-derived replicon that operates in *E. coli*.; rep(pUB 110) represents a pUB110-derived replicon that operates in *B. subtilis;* Kan^{R} represents a kanamycin resistance gene (aminoglycoside O-nucleotidyltransferase); and BJ27 promoter represents a strong promoter for *B. subtilis.*
FIG. 5 illustrates an overall procedure for preparing the production strain BSBA310.
FIG. 6 illustrates criteria for clinical outcome measurement used in the examples of the present disclosure.
FIG. 7 illustrates changes in total area of drusenoid lesions from the baseline according to the examples of the present disclosure. Compared with area change in the vehicle group, *p<0.05.
FIG. 8 illustrates changes in size of the largest drusenoid lesion from baseline according to the examples of the present disclosure.
FIGS. 9A and 9B illustrate results of blood biochemistry performed in the examples of the present disclosure.
FIGS. 10A to 10C illustrate results of blood hematology performed in the examples of the present disclosure.

### Best Mode for Carrying out Invention

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment to another embodiment or implemented in combinations of embodiments without departing from the technical spirit and scope of the present disclosure. Unless otherwise indicated, terms used in describing the present disclosure are to be understood in their ordinary meaning and apply to the same terms used herein as well as to the aspect or embodiment of the disclosure for which the term is defined. For purposes of interpreting this specification, the following definitions will apply and whenever appropriate, terms used in the singular will also include the plural and vice versa.

### Definition

As used herein, the term "about" refers to a typical margin of error for each value known to those skill in the art.

As used herein, the term "subject" is used interchangeably with "patient," and may be any mammal in need of prevention or treatment of dry macular degeneration, such as primate (for example, human), companion animal (for example, dog, cat and the like), domestic animal (for example, cow, pig, horse, sheep, goat and the like), and laboratory animal (for example, rat, mouse, guinea pig and the like). In an embodiment of the present disclosure, the subject is a human.

The term "treatment" includes prophylactic and/or therapeutic treatment. The prophylactic and/or therapeutic treatment includes all types of treatment recognized in the art and includes, for example, administering the pharmaceutical composition or SOD of the present disclosure to a subject. If it is administered prior to clinical manifestation of a unwanted or undesirable condition (for example, disease or other unwanted or undesirable state in the subject), the treatment is prophylactic treatment (for example, it protects the subject against developing the unwanted or undesirable condition). On the other hand, if it is administered after clinical manifestation of a unwanted or undesirable condition, the treatment is therapeutic treatment, such as to diminish, ameliorate, or stabilize the existing unwanted or undesirable condition or side effects thereof.

The meaning of the term "prevention" is well known in the art. When used in the context of a medical condition, such as dry macular degeneration, it means reducing frequency or delaying onset and symptoms of a medical condition (for example, blurred vision or vision loss) in a subject who has received the pharmaceutical composition or SOD of the present disclosure, as compared with a subject who did not receive the same.

The term "administration" refers to providing an active ingredient to a subject to achieve a prophylactic or therapeutic purpose (for example, prevention or treatment of dry macular degeneration).

### Dry macular degeneration

The present disclosure is based, at least in part, on the discovery that administration, in particular, oral administration, of a superoxide dismutase (SOD) is effective in preventing or treating dry macular degeneration. Therefore, according to an aspect of the present disclosure, there is provided a use of an SOD for preventing or treating dry macular degeneration.

The term "dry macular degeneration" is used interchangeably with "age-related dry macular degeneration." This includes all stages of dry macular degeneration: early, intermediate, and advanced.

Dry macular degeneration is characterized by drusen or drusenoid lesions. The term "drusen" refers to a product formed by accumulation of waste products, which are generated in the retina with age, between RPE cells and Bruch's membrane (B) or inside Bruch's membrane. Formation of drusen is a hallmark of dry-type AMD, and presence of multiple medium- and large-sized drusens is known to be a risk factor for progression to end-stage macular degeneration. In addition, the size and density of drusen are correlated with loss of choriocapillaris. Meanwhile, the term "drusenoid lesion" refers to a lesion caused by drusen.

The SOD of the present disclosure can effectively prevent, ameliorate, or treat dry macular degeneration by reducing drusen or drusenoid lesion or preventing formation thereof.

The present inventors conducted animal tests using rhesus monkeys, rather than mice, as an animal model that generates drusen with location and composition similar to a human. That is because, unlike transgenic mice, normal mice cannot be an effective model of dry AMD in that there is no evidence that light-induced oxidative stress affects retinal function (P. Sicard, et. al., "Dietary Superoxide Dismutase Protects Against Light- Induced Retinal Oxidative Stress in Young Senescence Accelerated Mice (SAM)," Invest Ophthalmol Vis Sci 2006;47: E-Abstract 2089), and drusen, which is a hallmark of dry AMD, is not produced (Hema L. Ramkumar, et. al. , "Retinal ultrastructure of murine models of dry age-related macular degeneration (AMD)," Progress In Retinal And Eye Research 2010;29(3), 169-190). In addition, according to the literature (Erica L. Fletcher, et. al., "Studying Age-Related Macular Degeneration Using Animal Models," Optom Vis Sci. 2014;91(8), 878-886), an anthropoid primate has been described to date as the only animal that develops drusen with composition and location similar to a human. Some mice develop white lesions with age; however, they are not an effective model for understanding formation of drusen because such lesions have different location and composition from a human.

### Superoxide dismutase (SOD)

According to another aspect of the present disclosure, there is provided an improved superoxide dismutase (SOD) that has an effect of preventing, ameliorating, or treating dry macular degeneration.

A superoxide dismutase (SOD) is an enzyme that alternately catalyzes dismutation of superoxide (O₂ ˙ ⁻) radical into either ordinary molecular oxygen (O₂) or hydrogen peroxide (H₂O₂). SODs play a key role in decreasing oxidative stress by removing reactive oxygen species. SODs are widely distributed in prokaryotic and eukaryotic cells and are classified into four classes based on their different types of metal centers (copper/zinc, nickel, manganese, and iron). Manganese-containing SODs (Mn-SODs) are widely present in many bacteria, chloroplasts, mitochondria, and cytosol of eukaryotic cells. The term "SOD" may be used interchangeably with a polypeptide having superoxide dismutase activity.

In an embodiment, the SOD may bind to manganese (Mn-SOD). Specifically, the SOD may be a deamidated Mn-SOD. More specifically, amino acid residues 73 and 136 of the SOD may be substituted with Asp residues, with respect to SEQ ID NO: 2. More specifically, the SOD may comprise or consist of the amino acid sequence represented by SEQ ID NO: 4.

The SOD or polypeptide having SOD activity of the present disclosure is interpreted to include amino acid sequences showing substantial identity to the above-mentioned amino acid sequence. The substantial identity means that in a case where aligned amino acid sequences are analyzed using an algorithm commonly used in the art, the sequences show sequence identity of 80% or higher, preferably 90% or higher, more preferably 95% or higher, and most preferably 98% or higher.

In another embodiment, the SOD may be a modified or engineered polypeptide having SOD enzymatic activity, and the polypeptide may comprise one or more mutations, for example, deletion, insertion, or substitution of one or more amino acids, which may or may not affect various aspects (in vivo, in vitro, or ex vivo stability, homogeneity, and/or conformational change). In addition, the polypeptide may further comprise a heterologous substance (for example, a tag known in the art, including HIS tag, HA tag, and myc tag, GFC, and/or Fc domain of an antibody) for purification, detection, or increased stability.

In an embodiment, the SOD may be derived from natural or recombinant microorganisms, and may be an enzyme produced through a process of isolation or purification from various sources.

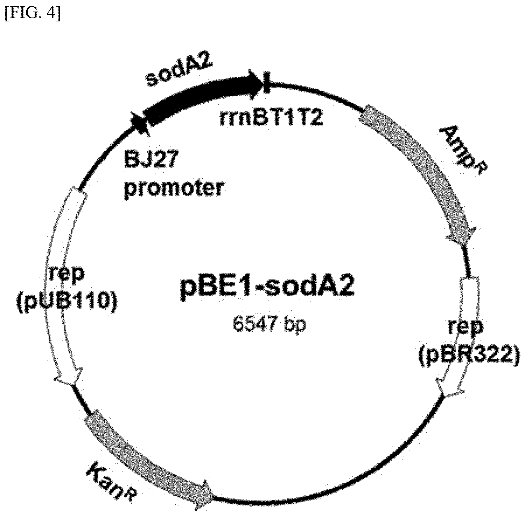

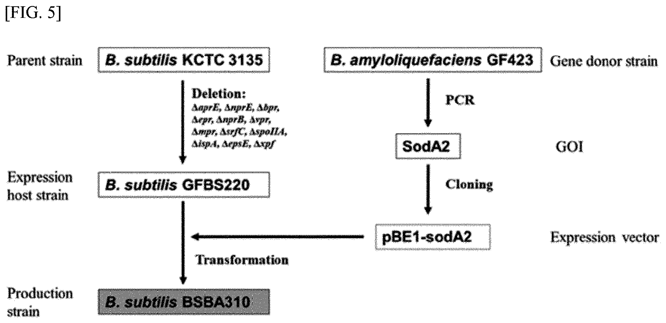

In an embodiment, the SOD may be derived from natural or recombinant microorganisms. For example, the SOD may be derived from bacteria. Preferably, the SOD may be derived from bacteria that are generally regarded as safe (GRAS) for use in drugs or foods. Specifically, the SOD may be derived from a Bacillus species strain. More specifically, the SOD may be derived from a *Bacillus amyloliquefaciens* strain. For example, the SOD may be derived from the *Bacillus amyloliquefaciens* strain GF423 (KCTC 13222 BP). The GF423 (KCTC 13222 BP) strain was deposited with the Korea Research Institute of Bioscience and Biotechnology on March 6, 2017. In addition, the SOD may be derived from a recombinant strain comprising the expression vector shown in FIG. 4. In addition, the SOD may be derived from a recombinant strain produced by a method including the method shown in FIG. 5. The recombinant strain may be a Bacillus species strain which comprises a nucleotide sequence encoding a polypeptide that comprises the amino acid sequence of SEQ ID NO: 4 and in which the following genes are deleted: *AprE, NprE, Bpr, Epr, NprB, Vpr, Mpr, IspA, SrfAC*, *spoIIAc*, *EpsE*, and *Xpf.* For detailed procedures for preparing the strain, see Example 1.

From the viewpoint that the SOD derived from the above-mentioned strain is an enzyme secreted extracellularly, in a case where an SOD is produced using the strain, it is possible to mass-produce an SOD, which is safe for humans, without going through an expensive purification process (for example, column purification), and this enables efficient production.

In an embodiment, the SOD may be isolated or purified from a variety of sources, including natural or recombinant hosts. For example, the SOD having activity of preventing or treating dry macular degeneration may be extracted from a culture supernatant of the *Bacillus amyloliquefaciens* strain GF423. Briefly, the Bacillus *amyloliquefaciens* strain GF423 may be first cultured in various types of media to obtain a culture. For example, the strain is grown at about 25°C to about 42°C for about 1 day to about 4 days using a complex medium (pH 6.0 to 7.0). Other suitable media for culturing the *Bacillus amyloliquefaciens* strain GF423 include Luria-Bertani (LB) medium, International Streptomyces Project (ISP) medium, nutrient agar (NA) medium, brain heart infusion agar (BHI) medium, sabouraud dextrose agar (SDA) medium, potato dextrose agar (PDA) medium, nutrient broth (NB) medium, and the like. Preferably, LB medium, ISP medium, BHI medium, SDA medium, or NB medium may be used. In addition, the SOD may be obtained from other natural or recombinant hosts using information provided in databases such as PubMed or BRENDA (www.brenda-enzymes.org).

In an embodiment, the SOD may be an isolated or purified enzyme. Here, the isolated or purified SOD, or a biologically active portion thereof is substantially free of cellular material or other contaminating proteins from the cell or tissue source from which it is derived. For example, the purified product may be separated purely from a strain culture through ultrafiltration, ammonium sulfate treatment, column purification, concentration, or the like, or may be a culture concentrate obtained therefrom through ultrafiltration, concentration, or the like. The phrase "substantially free of cellular material" includes preparations of a protein obtained by separating the protein from cellular components of the cell from which the protein is isolated or recombinantly produced. In an embodiment, the phrase "substantially free of cellular material" includes preparations of a protein in which unwanted proteins are present in an amount of less than about 30%, preferably less than about 20%, more preferably less than about 10%, and most preferably less than about 5% by dry weight.

In another embodiment, the SOD may be preferably purified by the following purification method, but the method is not limited thereto. The culture obtained by culturing the Bacillus amyloliquefaciens strain GF423 is centrifuged to collect a culture supernatant. The supernatant fraction is pretreated by solid-phase extraction, and then isolated and purified by chromatography. Here, various modes of chromatography may be used to purify the SOD. Preferably, hydrophobic interaction chromatography is used.

In another embodiment, the SOD may be included in a form of a strain lysate, strain culture, strain culture concentrate, or strain culture extract, or a dried form thereof. Here, the "strain lysate" may be obtained by culturing a strain and disrupting it mechanically or chemically. The "strain culture" may refer to a culture itself obtained by culturing a strain or a supernatant thereof. The "strain culture concentrate" refers to a product that is separated purely from a strain culture through ultrafiltration, ammonium sulfate treatment, column purification, concentration, or the like, or a culture concentrate obtained therefrom through ultrafiltration, concentration, or the like. The "strain culture extract" refers to a product that is extracted from the culture or a concentrate thereof, and may include an extract, a diluted or concentrated solution of the extract, a dried product obtained by drying the extract, or a crude or purified product thereof, or a fraction obtained by fractionating the same. The dried form may include a lyophilized form.

The SOD of the present disclosure may be used to reduce drusen or drusen lesions, and/or prevent formation thereof and/or effectively prevent, ameliorate, or treat dry macular degeneration. In particular, the SOD of the present disclosure is useful for preventing, ameliorating, or treating dry macular degeneration characterized by drusen or drusenoid lesions.

### Pharmaceutical composition

According to an aspect of the present disclosure, there is provided a pharmaceutical composition for treating or preventing dry macular degeneration, comprising a superoxide dismutase (SOD) as an active ingredient.

The pharmaceutical composition may reduce drusen or drusenoid lesions, or prevent formation thereof.

In an embodiment, the SOD according to the present disclosure may be combined with a pharmaceutically acceptable carrier, excipient and/or diluent to form a pharmaceutical composition for prevention or treatment of dry macular degeneration. The pharmaceutical composition of the present disclosure may be used interchangeably with the term veterinary composition when applied to an animal other than a human.

The pharmaceutical or veterinary composition of the present disclosure may further comprise a pharmaceutically acceptable carrier, excipient, and/or diluent. The pharmaceutically acceptable carrier, excipient, and/or diluent may be those commonly used in the art. The carrier, excipient, or diluent may include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, hydroxypropyl methyl cellulose, microcrystalline cellulose, polyvinyl pyrrolidone, water, methyl hydroxybenzoate, propyl hydroxybenzoate, propyl hydroxybenzoate, talc, magnesium stearate, silicon dioxide, and mineral oil, but are not limited thereto.

In a case of performing preparation, the preparation may be achieved by using an additive such as a filler, an extender, a binder, a wetting agent, a disintegrant, or a surfactant. The additive for the preparation may be appropriately selected from those commonly used in the pharmaceutical field.

In addition, the pharmaceutical or veterinary composition of the present disclosure may be formulated into a desired form depending on the method of use, and in particular, the formulation may be achieved by employing a method known in the art to provide rapid, sustained, or delayed release of the active ingredient after being administered to a mammal. Specific examples of such a formulation include tablet, pill, powder, granule, syrup, solution, capsule, suspension, emulsion, injection solution, plaster, lotion, liniment, lemonade, aerosol, extract, elixir, ointment, fluid extract, needle, cream, soft or hard gelatin capsule, patch, and the like.

Furthermore, the pharmaceutical or veterinary composition of the present disclosure may preferably be formulated using an appropriate method known in the art or a method disclosed in Remington's Pharmaceutical Science (latest edition), Mack Publishing Company, Easton PA.

In an embodiment, the SOD may be administered orally or parenterally. In a case of being orally administered, the SOD may be coated with shellac for protection from gastric acid, but the coating agent is not limited thereto. Examples of the coating agent suitable for use in the present disclosure include shellac, ethyl cellulose, hydroxypropyl methylcellulose, hydroxypropyl methylcellulose phthalate, zein, Eudragit, and combinations thereof. In a case where the SOD is coated, the SOD may be coated in solution. Specifically, a purified solution and a shellac-containing solution are mixed and then lyophilized. This lyophilized sample may be made into powder and stored at about 4°C until use. Solid preparations for oral administration include tablets, pills, powders, granules, capsules and the like, and these solid preparations may be prepared by mixing a composition with at least one excipient, such as starch, calcium carbonate, sucrose, lactose, gelatin and the like. In addition to simple excipients, a lubricant such as magnesium stearate and talc may also be used. Examples of liquid preparations for oral administration include suspensions, oral solutions, emulsions, and syrups. In addition to commonly used simple diluents such as water and liquid paraffin, various types of excipients, such as a wetting agent, a sweetener, a fragrance, and a preservative, may be used.

For parenteral administration, the administration may be achieved by an injection method selected from sublingual administration, intraocular administration including intravitreal administration, nasal spray, topical dermal application, patch, intraperitoneal injection, intrarectal injection, subcutaneous injection, intravenous injection, intramuscular injection, or intrathoracic injection.

In addition, the pharmaceutical or veterinary composition of the present disclosure is administered in a pharmaceutically or veterinary effective amount. The term "pharmaceutically effective amount" or "veterinary effective amount" refers to an amount sufficient to treat a disease at a reasonable benefit/risk ratio applicable to any medical or veterinary treatment. A level of the effective amount may be determined depending on factors including the patient's or animal's body weight, gender, age, health status, severity of disease, activity of the drug, sensitivity to the drug, administration time, administration route and excretion rate, duration of treatment, and drugs used concurrently therewith, and other factors well known in the medical field.

The pharmaceutical or veterinary composition of the present disclosure may be administered as an individual therapeutic agent or in combination with other therapeutic agents, in which the administration may be carried out in a sequential or simultaneous manner. In addition, the pharmaceutical composition may be administered once or multiple times as needed. Taking all of the above factors into consideration, it is important to administer a minimum amount that allows the maximum effect to be obtained with no adverse effects, and such an amount may be easily determined by those skilled in the art.

### Prophylactic or therapeutic method

According to yet another aspect of the present disclosure, there is provided a method for treating or preventing dry macular degeneration, comprising administering, to a subject having or at risk of dry macular degeneration, the SOD or pharmaceutical composition according to the present disclosure.

In addition, there is provided a method for treating or preventing dry macular degeneration, comprising administering to a subject a superoxide dismutase. The details regarding the SOD, dry macular degeneration, treatment, or prevention are the same as described above regarding the pharmaceutical composition.

The term "administration" includes any of a variety of administrations that accomplish a desired action and enable treatment. The administration may include oral or parenteral administration. For the oral or parenteral administration, see the details described above for the pharmaceutical composition. The route of administration may be oral, parenteral, inhalation, topical or localized (for example, intralesional) administration. For example, parenteral administration may include, but is not limited to, intravenous, subcutaneous, intraperitoneal, intraarterial, and intraocular administration. In a case of being orally administered, as described above, the SOD may be administered in a form coated with a coating agent such as shellac.

An effective amount or effective non-toxic amount of the SOD according to the present disclosure may be determined by conventional experimentation. For example, a therapeutically active amount of the SOD of the present disclosure may vary depending on factors such as disease stage, disease severity, the subject's age, gender, medical comorbidities, and body weight. Dosage and dosing regimen of the SOD of the present disclosure may be adjusted to provide an optimal therapeutic response. For example, several divided doses may be administered daily, weekly, every two weeks, every three weeks, every four weeks and the like, and/or the dose may be proportionally reduced or increased depending on exigencies of therapeutic situation.

In an embodiment, the method may include administering one or more additional agents for treating or preventing dry macular degeneration. The one or more additional agents may be administered simultaneously, sequentially, or in reverse order with the SOD or pharmaceutical composition. In addition, the individual agents may be administered to the subject by the same or different routes.

### Food composition

According to still yet another aspect of the present disclosure, there is provided a food composition for ameliorating or preventing dry macular degeneration, comprising the SOD according to the present disclosure as an active ingredient. Such a food composition includes a medical or nutraceutical food composition.

The term "medical food" or "nutraceutical food" refers to a food manufactured from raw materials or ingredients that are likely to have beneficial functions in the human body, the food maintaining normal function or activating physiological functions of the human body to maintain or improve health. This food is defined by the Ministry of Food and Drug Safety, but is not limited thereto. The food does not exclude any common health food from its meaning.

In addition, the food includes, but is not limited to, various foods, food additives, beverages (for example, functional drinks, natural fruit juices, and vegetable drinks), gum, tea, vitamin complexes, health functional foods, other functional foods, and the like.

The food may be manufactured by conventional methods known in the art. For example, the medical food, nutraceutical food, or health functional food may be formulated into one selected from the group consisting of tablet, pill, powder, granule, capsule, and liquid formulation by further comprising at least one of a carrier, a diluent, an excipient, and an additive, in addition to the SOD, for the purpose of preventing or ameliorating dry macular degeneration. Specific examples of the carrier, excipient, diluent, and additive are well known in the art, and those skilled in the art are capable of combining appropriate ingredients depending on the formulation to manufacture the food.

An amount of the superoxide dismutase according to the present disclosure as an active ingredient in the above-described formulation may be adjusted appropriately depending on the form and purpose of use, the patient's condition, type and severity of symptoms, and the like. The amount may be, but is not limited to, 0.001 to 99.9% by weight, and preferably 0.01 to 50% by weight, based on the weight of solid content.

A dose of the food of the present disclosure may vary depending on the patient's age, body weight, gender, dosage form, health status, and severity of disease, and the administration may be done once a day or in divided doses several times a day at regular time intervals depending on the judgment of a doctor or pharmacist. For example, the daily dosage may be 10 to 1,000 mg/kg based on the active ingredient content. The dosage is an example of an average case and may increase or decrease depending on individual differences. In a case where the daily dosage of the health functional food of the present disclosure is less than the above-mentioned dosage, it is not possible to obtain significant effects. In a case where the daily dosage exceeds the above-mentioned dosage, it is not only uneconomical but also undesirable side effects may occur because such a dosage is outside the usual dosage range.

### Feed composition

According to still yet another aspect of the present disclosure, there is provided a feed composition for preventing or ameliorating dry macular degeneration, comprising the SOD according to the present disclosure. In the present disclosure, the food composition may include a feed composition. This feed composition of the present disclosure may be prepared in any formulation commonly used in the art. For example, the feed composition of the present disclosure may further comprise an auxiliary ingredient such as amino acids, inorganic salts, vitamins, antibiotics, antibacterial substances, antioxidants, antifungal enzymes, and other microbial agents in the form of live bacteria; grain such as ground or broken wheat, oats, barley, corn, and rice; vegetable protein feed such as those comprising rapeseed, soybean, and sunflower as main ingredients; animal protein feed such as blood meal, meat meal, bone meal, and fish meal; a dry ingredient consisting of sugar and a dairy product such as various powdered milk and whey powder; lipid such as animal fat and vegetable fat, optionally liquefied by heating; and an additive such as nutritional supplements, digestion and absorption enhancers, growth promoters, and disease prevention agents.

The feed composition of the present disclosure may be in a form of a powder or liquid preparation, and may comprise an excipient that is added to feed. Excipients for feed additives include, but are not limited to, calcium carbonate, wheat shorts, zeolite, corn meal, rice bran, or the like.

### Polynucleotide, expression vector, and host cell

According to still yet another aspect of the present disclosure, there is provided a polynucleotide encoding the superoxide dismutase polypeptide. Specifically, there is provided a nucleic acid that encodes a polypeptide comprising or consisting of the amino acid sequence of SEQ ID NO: 4.

According to still yet another aspect of the present disclosure, there is provided an expression vector comprising the nucleic acid. Specifically, the vector may be the expression vector shown in FIG. 4.

According to still yet another aspect of the present disclosure, there is provided a host cell comprising the nucleic acid or expression vector. Specifically, the host cell is a bacterial cell. More specifically, the host cell is a Bacillus species strain.

According to still yet another aspect of the present disclosure, there is provided a Bacillus species strain which comprises the polynucleotide sequence that encodes a polypeptide comprising the amino acid sequence of SEQ ID NO: 4 and in which the following genes are deleted: *AprE, NprE, Bpr, Epr, NprB, Vpr, Mpr, IspA, SrfAC, spoIIAc, EpsE, and Xpf.* For the detailed procedures for preparing the strain, see Example 1 below.

Hereinafter, the present disclosure will be described in more detail by way of the following examples. The following examples are presented to help understand the present disclosure. These examples are not intended to limit a scope of the present disclosure in any way and should not be construed as limiting the present disclosure.

### Examples

### Example 1. Preparation of recombinant strain expressing superoxide dismutase (SodA2)

### Example 1.1. Preparation of expression host GFBS220

The expression host GFBS220 was prepared using *Bacillus subtilis* KCTC 3135. *B. subtilis* KCTC 3135 was obtained from the Korea Research Institute of Bioscience and Biotechnology, Biological Resources Center (KCTC). To facilitate post-processing, the genes shown in Table 1 below were removed from the genome of *B. subtilis* KCTC 3135.

**[Table 1]**

| Gene name | Protein name | Function |
|---|---|---|
| *AprE* | Serine alkaline protease (subtilisin E) | Extracellular protease |
| *NprE* | Extracellular neutral metalloprotease | Extracellular protease |
| *Bpr* | Bacillopeptidase F | Extracellular protease |
| *Epr* | Extracellular serine protease | Extracellular protease |
| *NprB* | Extracellular neutral protease B | Extracellular protease |
| *Vpr* | Extracellular serine protease | Extracellular protease |
| *Mpr* | Extracellular metalloprotease | Extracellular protease |
| *IspA* | Intracellular serine protease | Intracellular protease |
| *SrfAC* | Surfactin synthase | Surfactin synthesis |
| *spoIIAC* | RNA polymerase sporulation-specific sigma factor (sigma-F) | Sporulation |
| *EpsE* | Putative glycosyltransferase | Extracellular polysaccharide |
| *Xpf* | RNA polymerase sigma factor | Transcription of PBSX prophage gene |

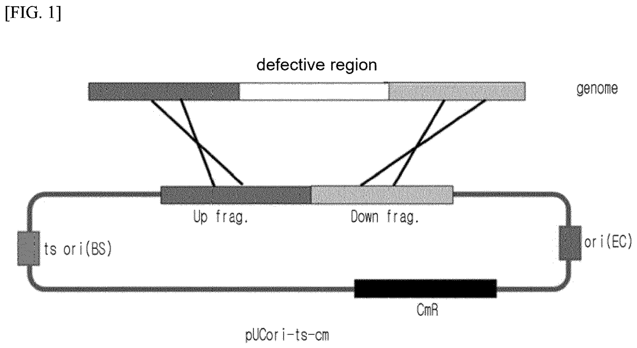

Removal of the genes was performed using double crossover recombination on the genome (FIG. 1). Specifically, DNA segments (up frag. and down frag.), which have homology to the DNA base sequences flanking the gene to be manipulated, were cloned into the vector pUCori-ts-cm that has a temperature-sensitive replication origin. PCR conditions and primers used for cloning are shown in Tables 2 and 3, respectively.

**[Table 2]**

| **PCR steps** | **Temperature** | **Time** |
|---|---|---|
| First denaturation | 95°C | 30 s |
| 30 cycles | 95°C | 30 s |
| | 50°C | 30 s |
| | 72°C | 1 min/kb |
| Final elongation | 72°C | 10 min |
| DNA polymerase used in PCR is *Taq* DNA polymerase (NEB, USA). | | |

**[Table 3]**

| Target gene | Homologous pair | | Primer sequence (5'→3') | SEQ ID NO |
|---|---|---|---|---|
| AprE | fragment 1 | F | ctacggggtctgacgcagatatcgtaaccgaagaacg | 5 |
| | | R | tgttgcagacatgttgctgaacgccatc | 6 |
| | fragment 2 | F | caacatgtctgcaacatatcttggaaac | 7 |
| | | R | gataagctgtcaaaccagagattggataggctatcaag | 8 |
| NprE | fragment 1 | F | ctacggggtctgacgcagggcattactgcaccataatc | 9 |
| | | R | gaggtacgccttcagcagcctgaacacc | 10 |
| | fragment 2 | F | gctgaaggcgtacctcacgccttcttcc | 11 |
| | | R | gataagctgtcaaaccagttgtcagatttgcatccttc | 12 |
| Bpr | fragment 1 | F | ctacggggtctgacgcagagcaatttccagcccgcttc | 13 |
| | | R | gtatgatgaggcatgaacagcaatcccg | 14 |
| | fragment 2 | F | tcatgcctcatcatactcaacaagggtg | 15 |
| | | R | gataagctgtcaaaccagttcccagccggatgttcaac | 16 |
| Epr | fragment 1 | F | ctacggggtctgacgcaggctttctgccagccgatagg | 17 |
| | | R | tgtgtcaaagccgttatgcttggctccg | 18 |
| | fragment 2 | F | taacggctttgacacagcacaaactgcc | 19 |
| | | R | gataagctgtcaaaccaggtctcccatacatgaacgac | 20 |
| NprB | fragment 1 | F | ctacggggtctgacgcagagtaatcttgtaggaggctg | 21 |
| | | R | tgtgactgtcatattcaccgtcgtgtgg | 22 |
| | fragment 2 | F | tgaatatgacagtcacacagtattccgcc | 23 |
| | | R | gataagctgtcaaaccagattaggatacggtctggctg | 24 |
| Vpr | fragment 1 | F | ctacggggtctgacgcagatgtacgctgagccgaatag | 25 |
| | | R | ggaatcacatttgcggagatacggcgtc | 26 |
| | fragment 2 | F | ccgcaaatgtgattccggcacatcaaac | 27 |
| | | R | gataagctgtcaaaccagaccgttttccgaatctgacc | 28 |
| Mpr | fragment 1 | F | ctacggggtctgacgcagcgttatcccgaatgcccgtg | 29 |
| | | R | ggctttgttcctttagtgtcaaccaccc | 30 |
| | fragment 2 | F | ctaaaggaacaaagccgattcgctccgc | 31 |
| | | R | gataagctgtcaaaccagaaattgactgctccacgctg | 32 |
| SrfAC | fragment 1 | F | ctacggggtctgacgcaggatatgtattacctatcgcc | 33 |
| | | R | gccccttcatccagttcactgcttccttg | 34 |
| | fragment 2 | F | ggaagcagtgaactggatgaaggggcttcgctg | 35 |
| | | R | gataagctgtcaaaccagaacgcttcgacatcactttc | 36 |
| spoIIA | fragment 1 | F | ctacggggtctgacgcagagcggacgatgggagactgg | 37 |
| | | R | gccacctcatgcagccgatctggaagag | 38 |
| | fragment 2 | F | ggctgcatgaggtggctgagcggctcgg | 39 |
| | | R | gataagctgtcaaaccagctcattgttttggtgagctg | 40 |
| IspA | fragment 1 | F | ctacggggtctgacgcagtgctgcttggcattcatttc | 41 |
| | | R | gccattgaagcaatgcctccgtttgaatc | 42 |
| | fragment 2 | F | acggaggcattgcttcaatggctgcccctcatg | 43 |
| | | R | gataagctgtcaaaccagtcaatgctctcgtcatattc | 44 |
| EpsE | fragment 1 | F | ctacggggtctgacgcagcaagcaaatgggctgggagg | 45 |
| | | R | gacaagccatgctttctgccaaagtgcg | 46 |
| | fragment 2 | F | gaaagcatggcttgtcaagcatgaatag | 47 |
| | | R | gataagctgtcaaaccagtgttgtatacattcagcagc | 48 |
| Xpf | fragment 1 | F | gatcctctacggttcatccatgtccgaac | 49 |
| | | R | tctatgatcctcctcatttttg | 50 |
| | fragment 2 | F | gaggaggatcatagaaagcttgtcatacgtttgcc | 51 |
| | | R | gagttttcgttcggatcctccgctttttgtttg | 52 |

The thus produced vectors were introduced into *B. subtilis* cells, and then transformants (single crossover homologous recombination) were selected using medium containing chloramphenicol at 37°C (non-replication temperature). The selected transformants were cultured (second crossover) at 30°C (replication-permissive temperature) in LB medium without antibiotics, and then cultured at 39°C in LB agar medium without antibiotics. Colonies susceptible to chloramphenicol were selected from the resulting colonies (plasmid curing). The selected colonies were subjected to PCR to obtain double crossover recombinants, and second pure separation was performed to select the final recombinant, which was named GFBS220.

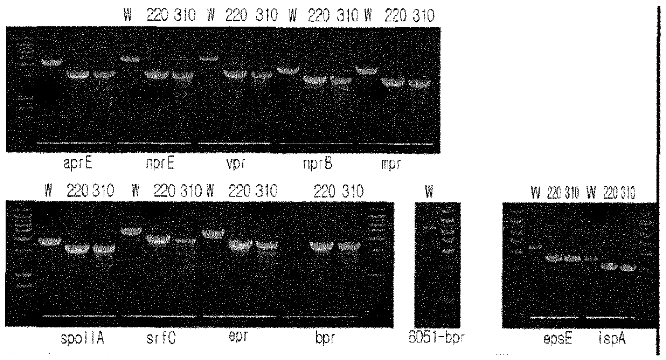

The defective gene regions of GFBS220 were amplified with the primers shown in Table 4 and PCR, and then identified using agarose gel electrophoresis. The results obtained by identifying the defective genes using PCR in the expression host GFBS220 are shown in FIG. 2.

**[Table 4]**

| Gene region (locus) | PCR primer (SEQ ID NO) | Size of PCR product of *B. subtilis* KCTC 3135 | Size of PCR product of GFBSL210 |
|---|---|---|---|
| *AprE* | F caaggcttgaaataacgttg (SEQ ID NO: 53) | 3115bp | 2116bp |
| | R ttcgctgattacaacattgg (SEQ ID NO: 54) | | |
| *NprE* | F ggaacagatgatagctcatc (SEQ ID NO: 55) | 3487bp | 2145bp |
| | R acactccgagaaggctgaag (SEQ ID NO: 56) | | |
| *Vpr* | F ctgcggcctgcacagccgcc (SEQ ID NO: 57) | 3733bp | 2293bp |
| | R cgctgaatgacggtggtaag (SEQ ID NO: 58) | | |
| *NprB* | F agtaatcttgtaggaggctg (SEQ ID NO: 59) | 2666bp | 2153bp |
| | R cgccaatgaagtgaaggagg (SEQ ID NO: 60) | | |
| *Mpr* | F ggttgaggcgattgcgacgg (SEQ ID NO: 61) | 2734bp | 2076bp |
| | R ccattctgcaaaatcagctc (SEQ ID NO: 62) | | |
| *spoIIAC* | F aagatatgaagaaagccggg (SEQ ID NO: 63) | 2628bp | 2102bp |
| | R acagccgcttcataagcctg (SEQ ID NO: 64) | | |
| *ThrC* | F tcaagctgtcatgtacggag (SEQ ID NO: 65) | 376bp | 5455bp |
| | R tccgatacgaatggctgtcg (SEQ ID NO: 66) | | |

### Example 1.2. Construction of expression vector pBE1-sodA2

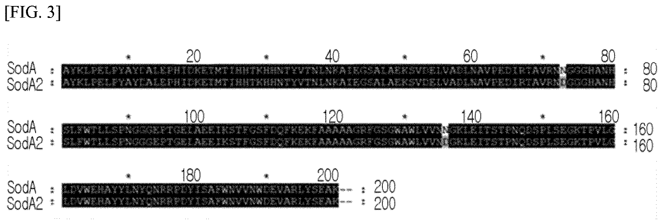

An Mn-SOD was identified in the culture supernatant of *Bacillus amyloliquesfaciens* strain GF423 through N-terminal sequencing (Kang et al., 2018). The GF423 strain was deposited with the Korea Research Institute of Bioscience and Biotechnology on March 6, 2017 (under accession number of KCTC 13222 BP). The gene of the Mn-SOD was named *sodA* according to the nomenclature for bacterial SOD. For the amino acid sequence thereof, see FIG. 3 and SEQ ID NO: 2 (for the nucleotide sequence thereof, see SEQ ID NO: 1). LC-UV-MS/MS peptide mapping of the enzyme preparation produced 100% amino acid sequence coverage; however, deamidation was observed at Asn73 and Asn136 residues at 73% and 17% abundance, respectively. Therefore, to improve homogeneity of the purified enzyme, both Asn residues were replaced with Asp residues. For the amino acid sequence of such variant sodA, named SodA2, see FIG. 3 and SEQ ID NO: 4 (for the nucleotide sequence thereof, see SEQ ID NO: 3).

The *sodA2* gene was amplified with duplicate elongation PCR using four primers (Table 5) and the genome of *Bacillus amyloliquefaciens* GF423 as a template. Nucleotides for replacement of Asn with Asp are underlined.

**[Table 5]**

| Primer | | Sequence (5'→3') | SEQ ID NO |
|---|---|---|---|
| 1 | SOD F | GGAGGAATTACAATGGCTTA CAA | 67 |
| 2 | D74 R | TGCATGTCCGCCGCCATCGT TGCGGAC | 68 |
| 3 | D74 F | CAGTCCGCAACGATGGCGGC GGACATG | 69 |
| 4 | D137 R | TTCAAGTTTGCCGTCGTTTAC AACGAGC | 70 |
| 5 | D137 F | CTCGTTGTAAACGACGGCAA ACTTG | 71 |
| 6 | SOD R | CAAAACAGAAGCTTCATTAT TTTGCT | 72 |

The amplified DNA fragment containing the *sodA2* gene and the plasmid pBE1 linearized by treatment with Ndel and HindIII were assembled *in vitro* by the SLIC method (Jeong et al. 2012), and then the resulting construct was transformed into *E. coli* C2984H. Correct clones were screened by restriction enzyme mapping and identified by nucleotide sequencing. The resulting expression vector pBE1-sodA2 is shown in FIG. 4.

### Example 1.3. Preparation of production strain BSBA310

The expression vector pBE1-sodA2 was transformed into *B. subtilis* GFBS220. Strains were selected from the transformants, and pure clones were established by two single colony isolation procedures in LB2 medium. The selected strain was named BSBA310 and stored at -80°C using the glycerol stock method. The results obtained by identifying the defective genes using PCR in this production strain BSBA310 are shown in FIG. 2. In addition, the overall procedure for preparing the production strain BSBA310 is summarized in FIG. 5.

### Example 2. Isolation and purification of superoxide dismutase (SodA2) from production strain BSBA310

### Example 2.1. Culture of production strain BSBA310

For culture of the production strain BSBA310 obtained in Example 1, the single colony formed on LB agar medium (LB (Luria-Bertani) agar; 10 g/L of tryptophan, 5 g/L of yeast extract, 10 g/L of NaCl, 15 g/L of agar) was inoculated into 30 ml of LB medium and cultured at 37°C for 12 hours. This seed culture was again inoculated into 3 L of LB medium containing 1 mM manganese sulfate (MnSO₄), and cultured at 37°C for 20 hours.

### Example 2.2. Isolation and purification of SodA2

The cell culture obtained in Example 2.1 was centrifuged 4 °C and 3,578 × g for 20 minutes to collect the supernatant, which was then concentrated 10-fold using ultrafiltration (hereinafter referred to as UF, MWCO 10,000). 390 g of ammonium sulfate was added thereto per liter of the concentrated cell culture, the mixture was stirred for 20 minutes, and centrifugation was performed to take the supernatant. The taken supernatant was purified using a phenyl Sepharose HP column. The purification process was as follows. The column was equilibrated using 50 mM potassium phosphate pH 7.0 with a concentration of 2 M ammonium sulfate. Then, the supernatant obtained by adding ammonium sulfate was passed through the column, and SodA2 attached to the column was recovered using 50 mM potassium phosphate pH 7.0 containing 1.6 M ammonium sulfate. The solution purified through the column was collected and concentrated by ultrafiltration while removing highly concentrated salts formed during the purification process. The concentrate was filtered through a sterilization filter and then lyophilized. Activity of SodA2 was analyzed using the SOD assay kit (Cayman Chemical, Michigan, USA). One unit of SOD activity is defined as an amount of the enzyme that inhibits superoxide radicals by 50%. Activity of the dried SodA2 enzyme ranged from 1000 to 1200 U/mg.

### Example 3. Preparation of SodA2 for animal test

### Preparation of material to be administered

Shellac (EXCELACS Co., Ltd., Bangkok) was dissolved in 100% ethanol to a concentration of 3% and a 0.2 µm sterile filter was prepared. Shellac solution dissolved in ethanol was diluted 1/20 times with sterile 1x PBS to prepare a shellac solution.

The lyophilized purified SodA2 was dissolved at 20 mg/ml and prepared to be sterile using the 0.2 µm filter.

The prepared shellac solution and the dissolved SOD were mixed in a 1:1 ratio with stirring. Then, stirring was continued for 10 minutes.

The well-stirred material was lyophilized. In this way, the final lyophilized shellac-coated SodA2 was prepared.

The lyophilized shellac-coated SodA2 was prepared by adding dextrin thereto at a ratio of 1:9 to 12 (shellac-coated SodA2:dextrin), and the final activity thereof was determined to be 90 to 110 U/mg through activity measurement.

### Example 4. Animal test

In this example, SodA2 prepared in Example 3 was orally administered to rhesus monkeys with spontaneous intermediate dry age-related macular degeneration (AMD) to evaluate safety and efficacy thereof.

Abbreviations used in this example are defined as follows:

| Abbreviation | Description |
|---|---|
| OD | Oculus Dexter |
| OS | Oculus Sinister |
| AMD | Age-related Macular Degeneration |
| RPE | Retinal Pigment Epithelium |
| FP | Fundus Photography |
| OCT | Optical Coherence Tomography |
| ROI | Region of interest |
| hr | Hour |
| kg | Kilogram |
| L | Liter |
| mg | Milligram |
| ml | Milliliter |
| min | Minute |
| NA | Not applicable |
| µg | Microgram |
| µL | Microliter |

### Example 4.1. Preparation of test material and control material

SodA2 and the vehicle administered to the control group had the following characteristics and conditions.

**[Table 6]**

| **Item** | **Lot number** | **Amount/Volu me** | **Storage condition** | **Physical Property** | **Activity unit** |
|---|---|---|---|---|---|
| SodA2 | 200605 | 24 g/vial | 2°C to 8°C protected from light | White-pink powder | 100 U/mg |
| vehicle for control group | 200605 | 20 g/vial | 2°C to 8°C protected from light | White-pink powder | NA |

### Example 4.2. Preparation of test animals

The rhesus monkeys used in this example were selected, supplied, and used based on the following criteria.

**[Table 7]**

| species | rhesus monkey |
|---|---|
| Vendor | Ya'an PriMed Breed Bio-tech Co., Ltd. |
| Number of monkeys | 1 male and 7 females |
| Body weight | 5.14 to 10.41 kg |
| Age | 17 to 25 years (equivalent to human age of 55 to 80 years using the generally accepted 3:1 ratio for human:rhesus age) |

### Inclusion criteria

Monkeys with at least one eye with intermediate dry AMD diagnosed using the AREDS classification for retinal drusen size and area (see AREDS classification in Table 8 for grading digital fundus photographs) on fundus photography which is characterized by any of the following features:
- Soft drusen and showing progression in drusen size or area within two years;
- Numerous drusens with at least one large drusen (grade 4, drusen diameter is > 116 µm);
- Total lesion area of drusenoid lesions is at least grade 4 (Grade 4, drusen area I-2 ≥ 0.089 mm²).

**[Table 8]**

| **Retinal finding** | **Grade code and regions evaluated** |
|---|---|
| **Drusen size (largest)** | 0: Absent |
| | 1: Questionable |
| | 2: Definite, diameter < C-0^{&} (≤58 µm) |
| | 3: Diameter > C-0, but < C-1 (59 to 116 µm) |
| | 4: Diameter > C-1, but < C-2 (117 to 233 µm) |
| | 5: Diameter > C-2 (≥234 µm) |
| | 8: Not gradable |
| **Drusen area^{#}** | 0: Area < C-0 |
| | 1: Area ≥ C-0, but < C-1 |
| | 2: Area ≥ C-1, but < C-2 |
| | 3: Area ≥ C-2, but < I-2 |
| | 4: Area ≥ I-2, but < O-2 |
| | 5: Area ≥ O-2, but < ½ DA |
| | 6: Area ≥ ½ DA, but < 1 DA |
| | 7: Area ≥ 1DA |
| | 8: Not gradable |
| # Area within modified Early Treatment of Diabetic Retinopathy Study (ETDRS) grid. | |
| The circular areas for grading measured in square millimeters are: C-0 = 0.003 mm², C-1 = 0.011 mm², C-2 = 0.043 mm², I-2 = 0.089 mm², O-2 = 0.297 mm², 1/2-disc area (DA) = 0.77 mm², 1 DA = 1.54 mm². | |

### Exclusion criteria

- Evidence or history of neovascular AMD or diabetic retinopathy [either eye];
- Severe heart failure (New York Heart Association grades III and IV) or left ventricular ejection fraction < 35% on any examination;
- Severe liver disease, ALT level increases > 3 times of the normal upper limit;
- Severe renal disease, with one of the following signs: (1) dialysis or eGFR < 20 ml/min/1.73 m²; (2) urinary protein/urinary creatinine is ≥ 1 g/g; (3) blood creatinine or albumin/urinary creatinine is ≥ 600 mg/g;
- Confounding ocular conditions such as aphakia; optic atrophy (optic disc pallor); any ocular condition that the investigator believes may be a confounding factor in this study.

The prepared monkeys were raised under the following conditions. All procedures in this experiment were following the Animal Welfare Act and were performed in a laboratory approved for laboratory animal welfare.

### Environmental conditions

Monkeys were housed in a temperature-controlled (18°C to 26°C) and humidity-controlled (40 % to 70 %) facility with a 12-hour light/dark cycle.

### Food intake

Each monkey was fed with 350 g of PriMed Diet every day. Food intake was assessed quantitatively every day.

### Water

All monkeys had ad libitum access to filtered tap water.

### Example 4.3. Test design

### Example 4.3.1. Group assignment

Totally 8 spontaneous intermediate dry AMD rhesus monkeys identified in recent 2 years were enrolled in the pre-study period (4 weeks) for baseline imaging examination. Among the 8 monkeys, retinal drusen area of 2 monkeys met grade 6 (area ≥ ½ DA, but < 1 DA), and 6 monkeys were between grade 4 (area ≥ I-2, but < O-2) and grade 5 (area ≥ O-2, but < ½. DA); and the monkeys were randomly divided into two groups with 4 monkeys in each group.

Baseline characteristics of the monkeys are presented in Table 9, and animal grouping and dosing are presented in Table 10.

**[Table 9]**

| Group | Monkey ID | Gender | Age | BW | Total drusen area (OD/ OS) | Largest lesion size (OD/ OS) | RPE thickness (OD/ OS) | RPE volume (OD/ OS) |
|---|---|---|---|---|---|---|---|---|
| | | | yrs | kg | mm² | mm | µm | mm³ |
| SodA2 2.5 mg/kg (n=4, 7 eyes) | 275 | Male | 25 | 10.41 | 0.810/1.310 | 0.429/0.461 | 32/33 | 0.83/0.88 |
| | 3702 | Female | 19 | 5.88 | 1.656/1.375 | 0.268/0.309 | 32/31 | 0.87/0.86 |
| | 5214 | Female | 22 | 7.59 | NA/2.692 | NA/0.234 | NA/36 | NA/1.00 |
| | 2026 | Female | 21 | 6.14 | 0.400/0.559 | 0.167/0.198 | 29/29 | 0.81/0.82 |
| | Mean±S D | / | 22± 3 | 7.51±2.08 | 1.257±0.781 | 0.295±0.11 3 | 32±2 | 0.87±0.06 |
| vehicle (n=4, 7 eyes) | 13116 | Female | 19 | 5.81 | 7.937/9.685 | 0.335/0.309 | 46/47 | 1.27/1.31 |
| | 1976 | Female | 17 | 6.16 | 1.012/0.761 | 0.223/0.229 | 31/33 | 0.82/0.86 |
| | 5170 | Female | 22 | 6.57 | 7.523/NA | 0.571/NA | 48/NA | 1.36/NA |
| | 5196 | Female | 22 | 7.56 | 1.214/1.221 | 0.257/0.320 | 30/30 | 0.81/0.81 |
| | Mean±S D | / | 20± 2 | 6.53±0.76 | 4.193±3.976 | 0.321±0.11 9 | 38±9 | 1.03±0.26 |

**[Table 10]**

| **Group** | **Number of monkeys** | **Number of enrolled eyes** | **Dosage** | **Frequency** | **Route of dosing** |
|---|---|---|---|---|---|
| Vehicle | 4 | 7 | 2.5 mg/kg | Once a dav | Oral administration |
| SodA2 | 4 | 7 | 2.5 mg/kg | Once a day | Oral administration |

### Example 4.3.2. Administration

SodA2 and vehicle were stuffed in a piece of dried kiwi fruit. Monkeys in the vehicle group and SodA2 group received oral administration of vehicle and SodA2 once a day for 4 months, respectively. SodA2 and the vehicle were prepared daily before oral administration.

### Example 4.3.3. Test procedures

### Observation of retinal drusen

Retinal drusen size and area (including change from baseline in retinal drusen size and area) of the test animals were measured by fundus photography (FP) on day 30 (D30), day 60 (D60), day 90 (D90), and day 120 (D120) after dosing. Each baseline was measured during the pre-study period.

Prior to image acquisition, the monkeys were anesthetized with intramuscular injection ofketamine:xylazine mix (1:1, 8 mg/kg ketamine). 2 drops oftropicamide phenylephrine eye drop were applied to each eye after anesthesia for pupil dilation. After being anesthetized, the monkeys were placed in a dark room until pupil diameter is greater than 6 mm. A self-retaining eyelid spectrum was placed in the eye.

FP images were acquired using a retinal camera (KOWA VX-20) according to the following procedure. The monkey's forehead was kept leaned against the forehead support. The working distance was adjusted and focused before taking the images. It was checked whether a macula centered image was obtained. The monkeys were cared according to related standard operating procedures (SOPs) during examination. The size and area of drusen were measured by two experienced image graders using Image J (Version 1.52, Wayne Rasband National Institutes of Health, USA). Drusenoid lesions and AMD were graded using the Age-Related Eye Disease Study 2 (AREDS2) system (Table 11). Grading involves a modified grid template adapted from the Early Treatment Diabetic Retinopathy Study (ETDRS), with a set of graduated circles used to estimate maximum drusen size and total area involved by pigment abnormalities and drusen. All measurements included the number of drusenoid lesions, total area of lesions, and size of largest lesion.

**[Table 11]**

| **Retinal finding** | **Grade code and area evaluated** |
|---|---|
| **Drusen size (maximum)** | 0: Absent |
| | 1: Questionable |
| | 2: Definite, diameter < C-0^{&} (≤58 µm) |
| | 3: Diameter > C-0, but < C-1 (59 to 116 µm) |
| | 4: Diameter > C-1, but < C-2 (117 to 233 µm) |
| | 5: Diameter > C-2 (≥234 µm) |
| | 8: Not gradable |
| **Drusen area^{#}** | 0: Area < C-0 |
| | 1: Area ≥ C-0, but < C-1 |
| | 2: Area ≥ C-1, but < C-2 |
| | 3: Area ≥ C-2, but < I-2 |
| | 4: Area ≥ I-2, but < O-2 |
| | 5: Area ≥ O-2, but < ½ DA |
| | 6: Area ≥ ½ DA, but < 1DA |
| | 7: Area ≥ 1DA |
| | 8: Not gradable |

### Cage-side observation

Each monkey was observed and physically examined once a day for changes of behavior, autonomic activity, mental performance, body surface color, excretion, and eye appearance.

### Food intake

Food intake was assessed qualitatively and assessed once a day during the study period.

### Body weight

After a 14-hour overnight fast, the monkeys were weighed using a scale during the pre-study period (baseline) and on D7, D14, D21, D30, D37, D45, D52, D60, D67, D75, D82, D90, D97, D105, D112, D120 after administration.

### Blood biochemistry examination

Equipment: Roche Cobas6000 Analyzer Series C501

### Sample Type: Plasma

Frequency: Baseline and D14, D30, D45, D60, D75, D90, D105, D120 after administration.

MDA and C3 were detected only at 3 time points (D90, D105, and D120). The parameters of blood biochemistry examination are presented in Table 12.

**[Table 12]**

| **Parameters** | **Code** | **Unit** | **Methods** |
|---|---|---|---|
| Fasting plasma glucose | FPG | mmol/L | UV. Enzymatic reference method with hexokinase |
| Total cholesterol | TC | mmol/L | Enzyme colorimetry, 1-point endpoint |
| Low-density lipoprotein | LDL | mmol/L | Homogenous enzyme colorimetric assay |
| High-density lipoprotein | HDL | mmol/L | Homogenous enzyme colorimetric assay |
| Triglyceride | TG | mmol/L | Homogenous enzyme colorimetric assay |
| Aspartate aminotransferase | AST | IU/L | Colorimetry |
| Alanine aminotransferase | ALT | IU/L | IFCC rate method |
| Total bilirubin | TBIL | µmol/L | Diazo method, 2-point endpoint |
| Direct bilirubin | DBIL | µmol/L | Diazo method, 2-point endpoint |
| Creatine phosphokinase | CK | g/L | Colorimetry, rate method |
| Urea nitrogen | BUN | mmol/L | Colorimetry, rate method |
| Creatinine | CR-S | µmol/L | Rate method |
| Alkaline phosphatase | ALP | IU/L | Colorimetry, rate method |
| Total protein | TP | g/L | Colorimetry, 2-point endpoint |
| Albumin | ALB | g/L | Colorimetrv, 2-point endpoint |
| γ-glutamyl transpeptidase | GGT | IU/L | Enzymatic colorimetry |
| High sensitivity C reactive protein | hs-CRP | mg/L | Particle-enhanced turbidimetric immunoassay |
| Cystatin C | CYSC | mg/L | Immunoturbidimetry |
| Malondialdehyde^{#} | MDA | ng/ml | Enzyme-linked immunosorbent assay |
| Complement fragment 3d^{#} | C3d | µg/ml | Enzyme-linked immunosorbent assay |

### Blood hematology examination

Equipment: Siemens ADVIA 212i/SYSMEX XN1000
Sample Type: Whole Blood
Frequency: Baseline and post-dose D14, D30, D45, D60, D75, D90, D105, D120.

Parameters of blood hematology tests are presented in Table 13.

**[Table 13]**

| **Parameters** | | **Code** | **Unit** | **Methods** |
|---|---|---|---|---|
| Red blood cells | | RBC | n × 10¹²/L | Laser beam method |
| Hemoglobin | | HGB | g/L | Colorimetric method with cyanmethemoglobin |
| Hematocrit | | HCT | % | Laser beam method |
| Average red blood cell hemoglobin concentration | | MCHC | g/L | Laser beam method |
| Average red blood cell volume | | MCV | fL | Laser beam method |
| Average red blood cell hemoglobin | | MCH | pg | Laser beam method |
| Red blood cell distribution width | | RDW | % | Laser beam method |
| Hemoglobin distribution width | | HDW | g/L | Laser beam method |
| Reticulocyte percent count | | Retic% | % | Laser beam method |
| Reticulocyte absolute count | | Retic# | n × 10^⁹/L | Laser beam method with colorimetric reaction for fluorescent staining of nucleic acids |
| Platelets | | PLT | n × 10⁹/L | Laser beam method |
| Mean platelet volume | | MPV | fL | Laser beam method |
| Platelet distribution width | | PDW | fl | Laser beam method |
| Platelet hematocrit | | PCT | % | Laser beam method |
| White blood cells | | WBC | n × 10⁹/L | Laser beam method |
| WBC differential percent count: | | | % | Laser beam method with colorimetric reaction for peroxiase staining |
| | neutrophils | NEUT% | | |
| | lymphocytes | LYMPH% | | |
| | eosinophils | EOS% | | |
| | basophils | BASO% | | |
| | monocvtes | MOMO% | | |
| WBC differential absolute count: | | | n × 10⁹/L | |
| | Neutrophils | NEUT | | |
| | Lymphocytes | LYMPH | | |
| | Eosinophils | EOS | | |
| | Basophils | BASO | | |
| | Monocvtes | MOMO | | |

### Data analysis

Data analysis consists of total area of drusenoid lesions, size of the largest drusenoid lesion, amount of drusenoid lesions, body weight, blood biochemistry, blood hematology, and the like.

Microsoft Office Excel 2013 and SPSS Statistics 23.0 were used for data processing and statistical analysis. The total area of drusenoid lesions, the size of the largest drusenoid lesion, and the amount of drusenoid lesions were analyzed by paired-sample T test. Differences were considered statistically significant when p values were <0.05.

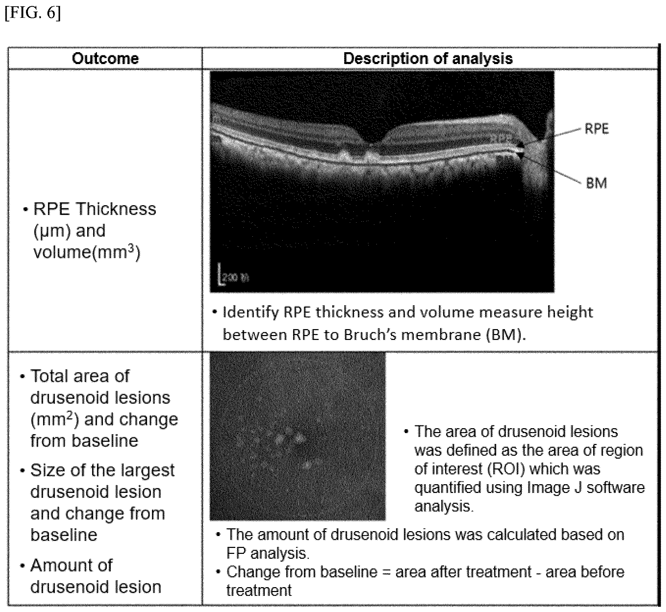

For criteria for clinical outcome measurement, see FIG. 6.

### Example 4.4. Results

### Example 4.4.1. Total area of drusenoid lesions

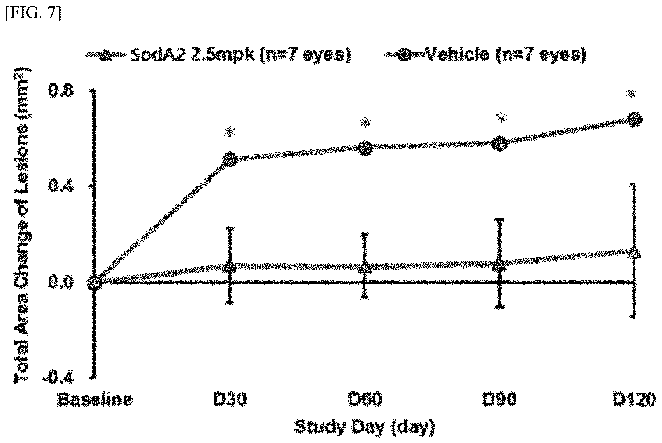

The total area of drusenoid lesions is shown in Table 14, and area changes are shown in Table 15 and FIG. 7.

In the 7 eyes of the vehicle group, the total area of drusenoid lesions had a significant increase at D30, D60, D90 and D120 after administration as compared with baseline (p<0.05). This suggests that the dry-AMD in the vehicle group obviously developed after 4-month administration with the vehicle.

In the 7 eyes of 2.5 mg/kg SodA2 group, the total area of drusenoid lesions did not change significantly at D30, D60, D90 and D120 after administration as compared with baseline. The change in total area of drusen lesions in the 2.5 mg/kg SodA2 group was significantly lower than the vehicle group (p<0.05). This suggests that SodA2 delayed progression of dry AMD.

**[Table 14]**

| **Group** | **Monkey ID-eye** | **Area (mm²)** | | | | |
|---|---|---|---|---|---|---|
| | | **Baseline** | **D30** | **D60** | **D90** | **D120** |
| **2.5 mg/kg SodA2 (n=7 eyes)** | 275 OD | 0.810 | 0.757 | 0.779 | 0.807 | 0.805 |
| | 275 OS | 1.310 | 1.280 | 1.222 | 1.252 | 1.285 |
| | 2026 OD | 0.400 | 0.457 | 0.443 | 0.451 | 0.541 |
| | 2026 OS | 0.559 | 0.642 | 0.654 | 0.626 | 0.717 |
| | 3702 OD | 1.656 | 1.871 | 1.884 | 1.963 | 2.268 |
| | 3702 OS | 1.375 | 1.698 | 1.622 | 1.708 | 1.670 |
| | 5214 OS | 2.692 | 2.578 | 2.663 | 2.532 | 2.432 |
| | Mean±SD | 1.257±0.781 | 1.326±0.769 | 1.324±0.788 | 1.334±0.768 | 1.388±0.760 |
| **Vehicle (n=77 eyes)** | 1976 OD | 1.012 | 0.840 | 0.958 | 0.961 | 1.052 |
| | 1976 OS | 0.761 | 0.779 | 0.750 | 0.778 | 0.820 |
| | 5170 OD | 7.523 | 9.225 | 9.090 | 9.058 | 9.571 |
| | 5196 OD | 1.214 | 1.518 | 1.498 | 1.550 | 1.584 |
| | 5196 OS | 1.221 | 1.455 | 1.341 | 1.486 | 1.541 |
| | 13116 OD | 7.937 | 8.644 | 9.141 | 9.097 | 9.051 |
| | 13116 OS | 9.685 | 10.478 | 10.504 | 10.486 | 10.508 |
| | Mean±SD | 4.193±3.976 | 4.706±4.479* | 4.755±4.542* | 4.774±4.498* | 4.875±4.550* |
| Compared with baseline, * p<0.05 | | | | | | |

**[Table 15]**

| **Group** | **Monkey ID-eye** | **Area change (mm²)** | | | | |
|---|---|---|---|---|---|---|
| | | **baseline** | **D30** | **D60** | **D90** | **D120** |
| **2.5 mg/kg SodA2 (n=7 eyes)** | 275 OD | 0.000 | -0.053 | -0.031 | -0.003 | -0.005 |
| | 275 OS | 0.000 | -0.030 | -0.088 | -0.058 | -0.025 |
| | 2026 OD | 0.000 | 0.057 | 0.043 | 0.051 | 0.141 |
| | 2026 OS | 0.000 | 0.083 | 0.095 | 0.067 | 0.158 |
| | 3702 OD | 0.000 | 0.215 | 0.228 | 0.307 | 0.612 |
| | 3702 OS | 0.000 | 0.323 | 0.247 | 0.333 | 0.295 |
| | 5214 OS | 0.000 | -0.114 | -0.029 | -0.160 | -0.260 |
| | Mean±SD | 0.000±0.000 | 0.069±0.155 * | 0.066±0.131* | 0.077±0.183* | 0.131±0.275 * |
| **Vehicle (n=7 eyes)** | 1976 OD | 0.000 | -0.172 | -0.054 | -0.051 | 0.040 |
| | 1976 OS | 0.000 | 0.018 | -0.011 | 0.017 | 0.059 |
| | 5170 OD | 0.000 | 1.702 | 1.567 | 1.535 | 2.048 |
| | 5196 OD | 0.000 | 0.304 | 0.284 | 0.336 | 0.370 |
| | 5196 OS | 0.000 | 0.234 | 0.120 | 0.265 | 0.320 |
| | 13116 OD | 0.000 | 0.707 | 1.204 | 1.160 | 1.114 |
| | 13116 OS | 0.000 | 0.793 | 0.819 | 0.801 | 0.823 |
| | Mean±SD | 0.000±0.000 | 0.512±0.628 | 0.561±0.641 | 0.580±0.602 | 0.682±0.719 |
| Area change = current area - baseline area; | | | | | | |
| Compared with area change of vehicle group, *p<0.05. | | | | | | |

### Example 4.4.2. Size of largest drusenoid lesions

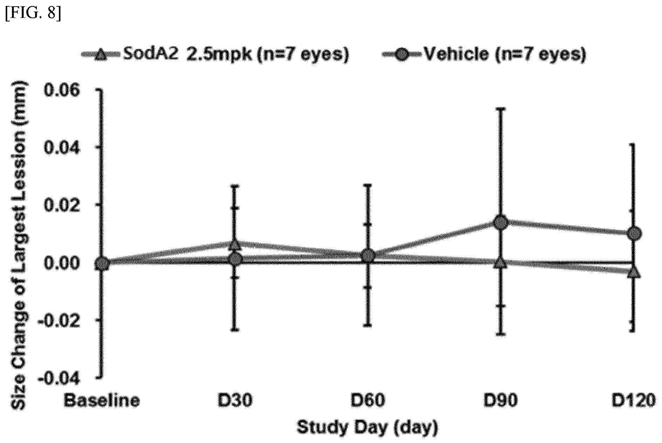

The size of the largest drusenoid lesion is shown in Table 16, and changes in size are shown in Table 17 and FIG. 8.

In the 7 eyes of the vehicle group, the size of the largest drusenoid lesion increased at D90 and D120, but there was no statistical significance compared with baseline. This suggests that progress of dry AMD in the vehicle group obviously developed after 4-month administration with the vehicle.

In the 7 eyes of the 2.5mg/kg SodA2 group, the size of the largest drusenoid lesion did not change significantly after administration compared with baseline. This suggests that 2.5 mg/kg SodA2 delayed progression of dry AMD.

**[Table 16]**

| **Group** | **Monkey ID-eye** | **Diameter (mm)** | | | | |
|---|---|---|---|---|---|---|
| | | **Baseline** | **D30** | **D60** | **D90** | **D120** |
| **2.5 mg/kg SodA2 (n=7 eyes)** | 275 OD | 0.429 | 0.428 | 0.422 | 0.426 | 0.424 |
| | 275 OS | 0.461 | 0.485 | 0.476 | 0.481 | 0.481 |
| | 2026 OD | 0.167 | 0.169 | 0.163 | 0.161 | 0.161 |
| | 2026 OS | 0.198 | 0.202 | 0.200 | 0.197 | 0.187 |
| | 3702 OD | 0.268 | 0.280 | 0.282 | 0.288 | 0.289 |
| | 3702 OS | 0.309 | 0.327 | 0.319 | 0.307 | 0.310 |
| | 5214 OS | 0.234 | 0.223 | 0.221 | 0.210 | 0.194 |
| | Mean±SD | 0.295±0.113 | 0.302±0.119 | 0.298±0.117 | 0.296±0.120 | 0.292±0.123 |
| **Vehicle (n=7 eyes)** | 1976 OD | 0.223 | 0.183 | 0.187 | 0.180 | 0.178 |
| | 1976 OS | 0.229 | 0.238 | 0.232 | 0.239 | 0.232 |
| | 5170 OD | 0.571 | 0.615 | 0.613 | 0.626 | 0.608 |
| | 5196 OD | 0.257 | 0.255 | 0.245 | 0.252 | 0.264 |
| | 5196 OS | 0.320 | 0.322 | 0.338 | 0.334 | 0.336 |
| | 13116 OD | 0.335 | 0.327 | 0.332 | 0.330 | 0.337 |
| | 13116 OS | 0.309 | 0.315 | 0.316 | 0.382 | 0.361 |
| | Mean±SD | 0.321±0.119 | 0.322±0.140 | 0.323±0.140 | 0.335±0.146 | 0.331±0.139 |

**[Table 17]**

| **Group** | **Monkey ID-eye** | **Size change (mm)** | | | | |
|---|---|---|---|---|---|---|
| | | **Baseline** | **D30** | **D60** | **D90** | **D120** |
| **2.5 mg/kg SodA2 (n=7 eyes)** | 275 OD | 0.000 | -0.001 | -0.007 | -0.003 | -0.005 |
| | 275 OS | 0.000 | 0.024 | 0.015 | 0.020 | 0.020 |
| | 2026 OD | 0.000 | 0.002 | -0.004 | -0.006 | -0.006 |
| | 2026 OS | 0.000 | 0.004 | 0.002 | -0.001 | -0.011 |
| | 3702 OD | 0.000 | 0.012 | 0.014 | 0.020 | 0.021 |
| | 3702 OS | 0.000 | 0.018 | 0.010 | -0.002 | 0.001 |
| | 5214 OS | 0.000 | -0.011 | -0.013 | -0.024 | -0.040 |
| | Mean±SD | 0.000±0.000 | 0.007±0.012 | 0.002±0.011 | 0.001±0.015 | -0.003±0.021 |
| **Vehicle (n=7 eyes)** | 1976 OD | 0.00 | -0.040 | -0.036 | -0.043 | -0.045 |
| | 1976 OS | 0.00 | 0.009 | 0.003 | 0.010 | 0.003 |
| | 5170 OD | 0.00 | 0.044 | 0.042 | 0.055 | 0.037 |
| | 5196 OD | 0.00 | -0.002 | -0.012 | -0.005 | 0.007 |
| | 5196 OS | 0.00 | 0.002 | 0.018 | 0.014 | 0.016 |
| | 13116 OD | 0.00 | -0.008 | -0.003 | -0.005 | 0.002 |
| | 13116 OS | 0.00 | 0.006 | 0.007 | 0.073 | 0.052 |
| | Mean±SD | 0.000±0.000 | 0.002±0.025 | 0.003±0.024 | 0.014±0.039 | 0.010±0.031 |
| Size change = current size - baseline size | | | | | | |

### Example 4.4.3. Cage-side observation

No significant abnormalities were found in behavior, autonomic activity, skin, hair, excretion, and eyes of the monkeys in each group before and after administration. No toxicity-related issues were found in this study.

### Example 4.4.4. Body weight

No obvious changes were found in body weight for the monkeys in each group during the administration period. The relevant results are presented in Table 18 where body weight is in kg (mean ± SD).

**[Table 18]**

| Study date | 2.5 mg/kg SodA2 (n=4) | Vehicle (n=4) |
|---|---|---|
| Baseline | 7.51±2.08 | 6.53±0.76 |
| D7 | 7.51±1.97 | 6.58±0.59 |
| D14 | 7.60±2.08 | 6.60±0.45 |
| D22 | 7.61±2.12 | 6.71±0.61 |
| D30 | 7.68±2.37 | 6.71±0.61 |
| D37 | 7.58±2.28 | 6.62±0.56 |
| D45 | 7.73±2.44 | 6.56±0.44 |
| D52 | 7.86±2.59 | 6.67±0.55 |
| D60 | 7.85±2.55 | 6.69±0.68 |
| D67 | 7.64±2.39 | 6.62±0.46 |
| D75 | 7.72±2.40 | 6.62±0.49 |
| D82 | 7.83±2.60 | 6.67±0.40 |
| D90 | 7.96±2.82 | 6.69±0.44 |
| D97 | 7.94±2.79 | 6.71±0.37 |
| D105 | 8.03±2.88 | 6.62±0.44 |
| D112 | 8.12±2.96 | 6.86±0.43 |
| D120 | 8.07±2.82 | 6.82±0.43 |

### Example 4.4.5. Blood biochemistry

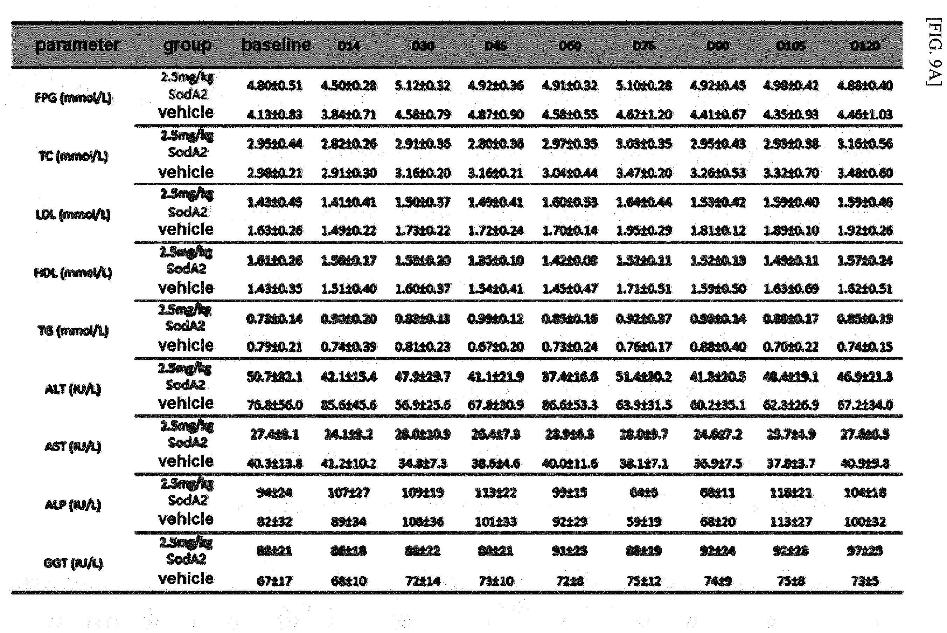

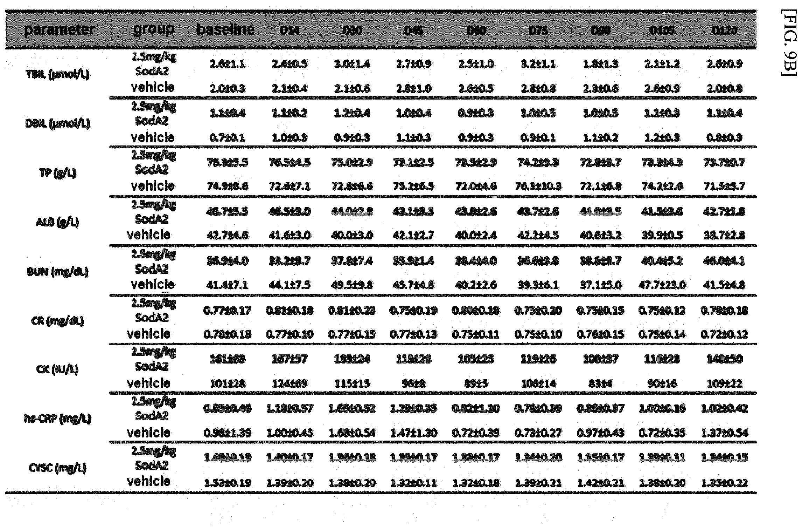

No obvious changes were found in blood biochemistry for the monkeys in each group during the administration period. The relevant results are presented in FIG. 9 (expressed as mean ± SD).

### Example 4.4.6. Blood hematology

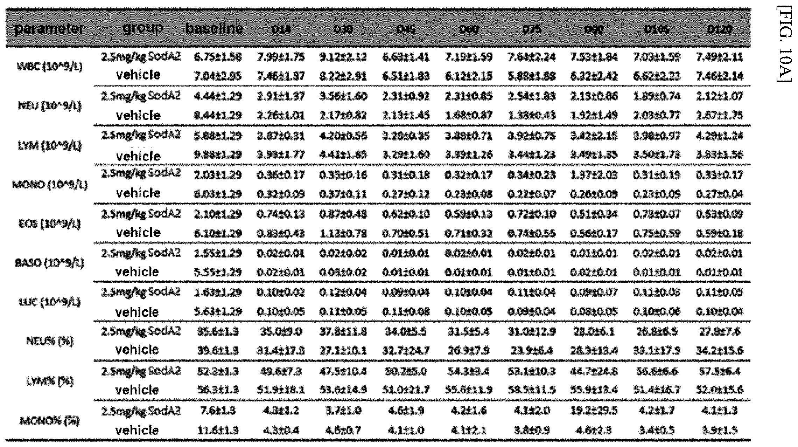

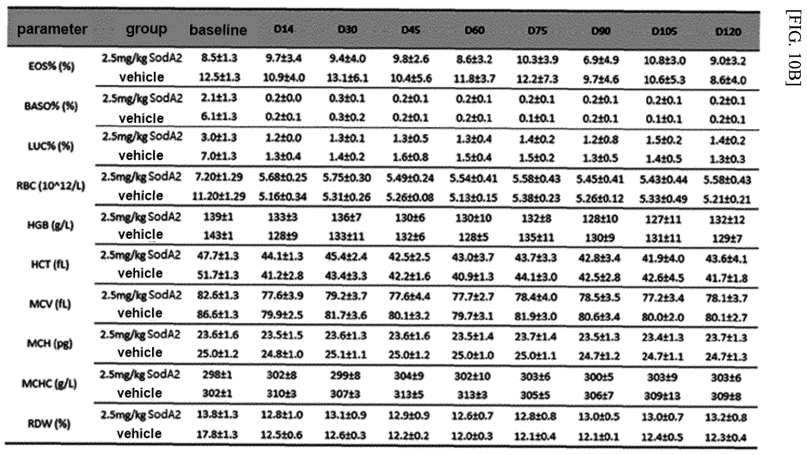

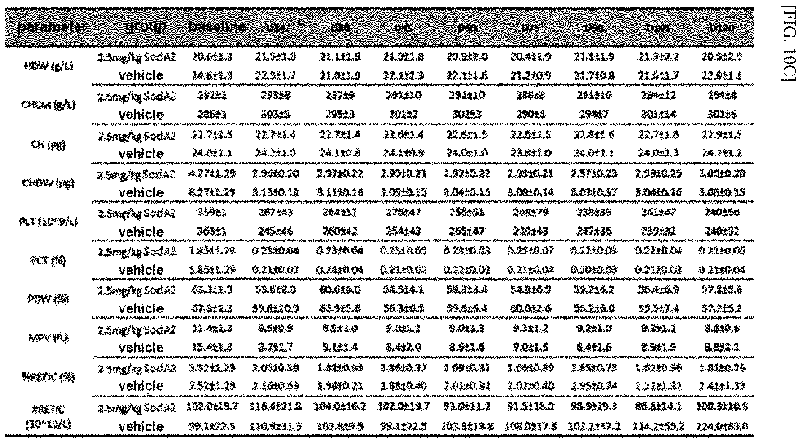

No obvious changes were found in blood hematology for the monkeys in each group during the administration period. The relevant results are presented in FIG. 10 (expressed as mean ± SD).

### Example 4.4.7. Conclusion

In the 7 eyes of the vehicle group, the total area of drusenoid lesions increased significantly at D30, D60, D90 and D120 after administration compared with baseline (p<0.05); the size of the largest drusenoid lesion increased at D90 and D120, but no statistical significance was found compared with baseline This suggests that the drusenoid lesions developed in the monkeys of the vehicle group that received 4-month administration of the vehicle.

In the 7 eyes of the 2.5 mg/kg SodA2 group, the total area of drusenoid lesions did not change significantly after administration compared with baseline; the change in total area of drusenoid lesions was significantly lower than the vehicle group (p<0.05); and the size of the largest drusenoid lesion did not change significantly after administration compared with baseline, and showed a tendency to decrease. This suggests that 2.5 mg/kg SodA2 delayed progression of drusenoid lesions.

Meanwhile, no obvious changes were found in body weight, blood biochemistry, and hematology of monkeys in each group during 4-month administration. No toxicity-related issues were found in this study. In conclusion, oral administration of SodA2 can delay progression of drusenoid lesions in dry AMD without toxicity-related issues.

## Claims

1. A pharmaceutical composition for treating or preventing dry macular degeneration, comprising a superoxide dismutase (SOD) as an active ingredient.

2. The pharmaceutical composition of claim 1, wherein the composition reduces drusen or drusenoid lesion or prevents formation thereof.

3. The pharmaceutical composition of claim 1, wherein the SOD is an isolated or purified enzyme.

4. The pharmaceutical composition of claim 1, wherein the SOD binds manganese.

5. The pharmaceutical composition of claim 1, wherein the SOD is a deamidated Mn-SOD.

6. The pharmaceutical composition of claim 1, wherein the SOD is derived from a Bacillus species strain.

7. The pharmaceutical composition of claim 1, wherein the SOD is derived from *Bacillus amyloliquefaciens* GF423 strain (KCTC 13222BP).

8. The pharmaceutical composition of claim 1, wherein amino acid residues 73 and 136 of the SOD are substituted with Asp residues, with respect to SEQ ID NO: 2.

9. The pharmaceutical composition of claim 1, wherein the SOD has an amino acid sequence as set forth in SEQ ID NO: 4.

10. The pharmaceutical composition of claim 1, wherein the SOD is administered orally or parenterally.

11. A veterinary composition for treating or preventing dry macular degeneration, comprising a superoxide dismutase (SOD) as an active ingredient.

12. A food composition for ameliorating or preventing dry macular degeneration, comprising a superoxide dismutase (SOD) as an active ingredient.

13. The food composition of claim 12, wherein the SOD is an isolated or purified enzyme.

14. The food composition of claim 12, wherein the SOD is included in a form of a strain culture or a strain culture concentrate, or a dried form thereof.

15. The food composition of claim 12, wherein the SOD binds manganese.

16. The food composition of claim 12, wherein the SOD is a deamidated Mn-SOD.

17. The food composition of claim 12, wherein the SOD is derived from a Bacillus species strain.

18. The food composition of claim 12, wherein the SOD is derived from *Bacillus amyloliquefaciens.*

19. The food composition of claim 12, wherein amino acid residues 73 and 136 the SOD are substituted with Asp residues, with respect to SEQ ID NO: 2.

20. A feed composition for ameliorating or preventing dry macular degeneration, comprising a superoxide dismutase (SOD) as an active ingredient.

21. A method for treating or preventing dry macular degeneration, comprising administering to a subject the pharmaceutical composition of any one of claims 1 to 10 or a superoxide dismutase (SOD).

22. The method of claim 21, wherein the administration of the SOD reduces drusen or drusenoid lesion or prevents formation thereof in the subject.

23. The method of claim 21, wherein the SOD is administered orally or parenterally.

24. A superoxide dismutase, which is derived from a Bacillus species strain and whose amino acid residues 73 and 136 are substituted with Asp residues, with respect to SEQ ID NO: 2.

25. The superoxide dismutase of claim 24, wherein the superoxide dismutase consists of an amino acid sequence as set forth in SEQ ID NO: 4.

26. A polynucleotide, encoding the superoxide dismutase polypeptide of claim 24 or 25.

27. A Bacillus species strain, which comprises a nucleotide sequence encoding a polypeptide comprising an amino acid sequence whose amino acid residues 73 and 136 are substituted with Asp residues, with respect to SEQ ID NO: 2, and in which the following genes are deleted:
AprE, NprE, Bpr, Epr, NprB, Vpr, Mpr, IspA, SrfAC, spoIIAc, EpsE, and Xpf.

28. A recombinant strain, comprising an expression vector as illustrated in FIG. 4.

29. A use of the pharmaceutical composition of any one of claims 1 to 10 or a superoxide dismutase (SOD), for preventing or treating dry macular degeneration.

30. A use of the pharmaceutical composition of any one of claims 1 to 10 or a superoxide dismutase (SOD), in manufacture of a medicament for prevention or treatment of dry macular degeneration.
